## Europäisches Patentamt

## European Patent Office

## Office européen des brevets

(19)

(11) Publication number: **0 080 440**
**A1**

(12) # EUROPEAN PATENT APPLICATION

(21) Application number: **82830284.4**

(22) Date of filing: **17.11.82**

(51) Int. Cl.³: **A 61 C 15/00**

(30) Priority: **23.11.81 IT 4975881**

(43) Date of publication of application: **01.06.83**
**Bulletin 83/22**

(84) Designated Contracting States: **DE FR GB SE**

(71) Applicant: **Minozzi, Romualdo, Via Pitocrito 4,**
**I-00124 Roma (IT)**

(72) Inventor: **Minozzi, Romualdo, Via Pitocrito 4,**
**I-00124 Roma (IT)**

(74) Representative: **Massari, Marcello, Studio M. Massari 23,**
**Via della Fontanella Borghese, I-00186 Roma (IT)**

(54) **A dental floss performing a disinfecting action and/or a preventive action against bacterial plaques and/or caries.**

(57) A dental floss, which is normally used in dentistry only for a mechanical cleaning of the spaces between the teeth, is given a disinfecting and therapeutic-prophylactic property with a specific disinfecting and preventive action against bacterial plaques and caries obtained by forming a film of a substance containing the suitable agents on the surface of the dental floss, these agents being preferably one or more of the following active principles each having the above-mentioned properties:
— quaternary ammonium bases performing a disinfecting action;
— chloroexidine digluconate performing a preventive action against bacterial plaques;
— sodium mono-fluorophosphate performing a preventive action against caries;
this is preferably otbained by locating the reel (10) of dental floss (11) within a case in the open upper part (22) of which a closed chamber (14) is received containing a mass of a gel including the above agents; the closed chamber has an opening (19, 20) both in the top and bottom walls (16, 18) through which the dental floss (11) passes through the chamber (14) and the gel mass that, consequently, forms the film on the dental floss (10) as the same is unwound from reel (10).

ACTORUM AG

1

# A DENTAL FLOSS PERFORMING A DISINFECTING ACTION AND/OR A PREVENTIVE ACTION AGAINST BACTERIAL PLAQUES AND/OR CARIES

This invention relates to a dental floss of the type normally used in dentistry only for a mechanical cleaning action of the spaces between the teeth, which is also given a disinfecting and therapeutic-prophylatic property with a specific disinfecting and preventive action against bacterial plaques and caries. This is obtained by forming a film of a substance containing one or more active principles, each having the above mentioned property, in any suitable way, on the dental floss surface. The principles can be a) quaternary ammonium bases for the disinfecting action; b) chloroexidine digluconate for the preventive action against bacterial plaques; c) sodium mono-fluorophosphate or other fluorine compounds for the preventive action against caries. At present, trimethylcetyl ammonium para-toluenesulfonate is the quaternary ammonium base preferred as an antiseptic product. According to the invention, the film of active substance can be formed either during the manufacture of the dental floss or by placing a reel thereof dipped into a gel containing the active principles within a case, or preferably, upon using the dental floss as it is drawn out of its case. In other words, according to a preferred embodiment of the invention the untreated dental floss is received within a case in the upper portion of which there is placed a mass of a gel containing the above-mentioned active principles. This gel mass is received within a chamber suitably fitted into the opening of the case containing the dental floss reel.

Suitable openings are formed both on the chamber bottom, which separates the chamber from the space containing the dental floss and in the chamber upper wall which separates the chamber from the atmosphere. The dental floss end to be unwound is passed through the opening on the chamber bottom, the gel mass and the opening in the chamber upper wall. The dental floss is then drawn out of the case, the end of which is closed by a suitable screw cap outside the chamber upper wall. It should be noted that the two openings are preferably offset on opposite sides with respect to the chamber axis. Accordingly, the dental floss path within the gel is longer to help the formation of the gel film on the dental floss surface.

The gel composition preferred at present is as follows:

| | |
|---|---|
| carboxymethylcellulose | 1% |
| glycerol | 50% |
| micronized silica | 5% |
| sorbitol | 10% |
| xylitol | 10% |
| water | as necessary. |

Furthermore, the percentages in weight of the above-mentioned active principles relative to the gel are preferably comprised within the following ranges:

| | | |
|---|---|---|
| trimethylcetyl ammonium para-toluenesulfonate | 0,050 + | 3% |
| chloroexidine digluconate | 0,020 + | 0,1% |
| sodium mono-fluorophosphate | 0,05 + | 0,15% |

The invention will be now described in detail with reference to the annexed drawing which is an exploded sectional view showing a case according to a presently preferred embodiment of the invention.

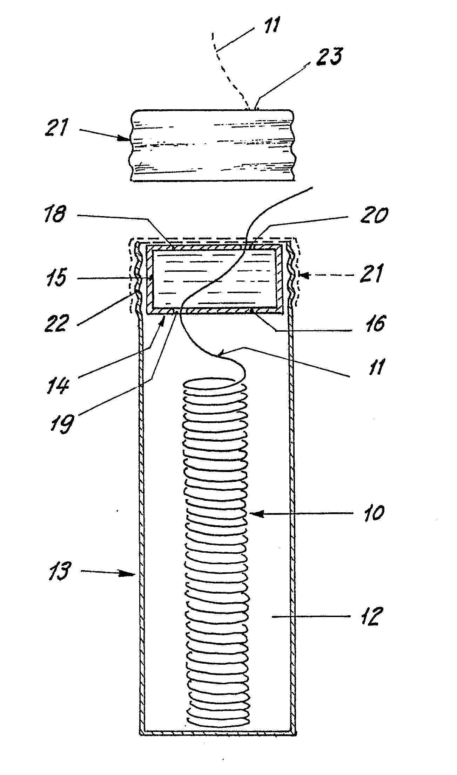

With reference to the drawing, in a presently preferred embodiment of the invention reel 10 of dental floss 11 is received within cavity 12 of a cylindrical case 13. A closed cylindrical chamber referred to by numeral 14 is fitted into the upper portion of case 13. Chamber 14 comprises a side wall 15, a bottom 16 and an upper wall 18. A small slot 19 is formed on bottom 16, which slot 19 is offset with respect to the chamber axis, and a small hole 20 is formed in upper wall 18. Hole 20 is offset relative to opening 19, thus being placed on the opposite side of the chamber axis.

The upper opening of case 13 which receives chamber 14 is closed by a screw cap referred to by 21. Cap 21 is threadingly engaged with the correspondingly threaded end 22 of case 13 and is provided with at least one hole referred to by numeral 23 through which dental floss 11 can be drawn out of case 13.

Chamber 14 receives the gel containing the active principles according to the invention, the preferred composition of which will be described below.

As it will be evident from the foregoing, the length of dental floss 11 which is unwound from reel 10 is first passed through slot 19 and then through hole 20, thus having an oblique path through the gel-containing chamber 14.

Accordingly, as the dental floss is drawn out of the upper portion of cap 21 to be used it is covered with a film of the gel contained within chamber 14 through which the dental floss passes.

In all the possible embodiments of the invention the composition of the gel to be associated with the dental floss in order to allow the dental floss to perform the above-mentioned therapeutic and prophylactic action besides the mechanical action thereof, is as follows:

| | |
|---|---|
| carboxymethylcellulose | 1% |
| glycerol | 50% |
| micronized silica | 5% |
| sorbitol | 10% |
| xylitol | 10% |
| trimethylcetyl ammonium para-toluenesulfonate | 0,1% |
| chloroexidine digluconate | 0,025% |
| sodium mono-fluorophosphate | 0,1% |
| water | as necessary. |

It should be understood that the invention is not limited either to the foregoing or to the above-mentioned substances and percentages thereof, the invention comprising, however, a dental floss associated with one or more substances carried thereby to support and complete the simply mechanical action performed by the dental floss between the teeth owing to their antiseptic and preventive action against bacterial plaques and **caries**.

The above-mentioned quaternary ammonium base, for example, can be replaced by cetylpyridinium chloride which performs a similar antiseptic action; chloroexidine digluconate can be replaced by benzalkonium chloride which has a similar preventive action against bacterial plaques and the like.

0080440

- 7 -

CLAIMS

1.- A dental floss associated with one or more active substances carried thereby and intended to support and complete the simply mechanical action performed by said dental floss owing to their antiseptic and preventive action against bacterial plaques and caries.

2.- The dental floss according to claim 1, wherein said active substance and substances having an antiseptic and preventive property against bacterial plaques and caries are contained in a gel forming a film on the surface of said dental floss.

3.-The dental floss according to claims 1 and 2, wherein the active substance intended to perform the antiseptic action is a quaternary ammonium base, the preventive substance against bacterial plaques is chloroexidine digluconate and the preventive substance against caries is sodium mono-fluorophosphate.

4.- The dental floss according to claim 3, wherein said quaternary ammonium base is trimethylcetyl ammonium para-toluenesulfonate.

5.- The dental floss according to claim 4, wherein the percentages of said active substances relative to said gel are comprised within the following ranges:

| | | |
|---|---|---|
| trimethylcetyl ammonium | | |
| para-toluenesulfonate | 0,05 | + 3% |
| chloroexidine digluconate | 0,02 | + 1% |

sodium mono-fluorophosphate     0,05   +   0,15%.

6.- The dental floss according to claim 5, wherein said gel preferably contains said active substances in the following percentages:

trimethylcetyl ammonium

para-toluenesulfonate               0,1%

chloroexidine digluconate           0,025%

sodium mono-fluorophosphate         0,1%.

7.- The dental floss according to claim 2 received within a case closed at one end and opened at the other end, provided with a screw cap, a closed chamber being fitted within said open end of said case, said closed chamber receiving a gel containing said active substances and having an opening in the top and bottom wall thereof, said dental floss being passed through said chamber and consequently the gel received therein thus becoming covered with a film of said gel as the latter is drawn out of said case to be used.

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| | --- | | A 61 C 15/00 |
| X | US-A-3 830 247 (P.KAPHALAKOS) *Claims 1-3; figure 1* | 1,2,7 | |
| Y | | 3 | |
| X | US-A-3 942 539 (J.J.CORLISS et al.) *Column 1, lines 59-66; claim 1* | 1,2 | |
| Y | | 3,5 | |
| Y | GB-A-2 002 830 (JANAR) *Claims 1,6* | 3,5 | |
| Y | CHEMICAL ABSTRACTS, vol. 85, no. 4, 26th July 1976, page 257, no. 25383v, Columbus Ohio (USA); & SE - A - 380 434 (B.MOGARD) (10-11-1975) *Abstract* | 3 | TECHNICAL FIELDS SEARCHED (Int. Cl. 3) A 61 C 15/00 |
| Y | DE-A-2 706 199 (M.J.E.WHITE) *Page 6, line 2; page 7; lines 12-15* | 3,5 | |
| Y | US-A-4 110 429 (A.GAFFAR et al.) *Column 1, lines 26-45; column 7, lines 19-35; claims 1-16* | 3,4,5 | |
| | --- -/- | | |

The present search report has been drawn up for all claims

| Place of search | Date of completion of the search | Examiner |
|---|---|---|
| THE HAGUE | 17-02-1983 | PELTRE CHR. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

## European Patent Office

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US-A-4 019 522 (C.H.ELBREDER) <br> --- | 1,7 | |
| P | CHEMICAL ABSTRACTS, vol. 97, no. 2, 12th July 1982, page 352, no. 11651s, Columbus Ohio (USA); & JP - A - 82 46 912 (SUNSTAR INC.)(17-03-1982) *Abstract* <br> ----- | 1-3 | |
| | | | **TECHNICAL FIELDS SEARCHED (Int. Cl. 3)** |

The present search report has been drawn up for all claims

| Place of search THE HAGUE | Date of completion of the search 17-02-1983 | Examiner PELTRE CHR. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82